# FASCICULE DE BREVET EUROPEEN

(11) **EP 3 518 249 B1**
(45) Date de publication et mention de la délivrance du brevet: **16.03.2022**
(21) Numéro de dépôt: 18154284.6
(22) Date de dépôt: 30.01.2018
(51) Int. Cl.: G16H 50/70, G16H 50/20, A61C 7/00, A61C 13/00, G16H 30/40

(54) **PROCÉDÉ D'ANALYSE D'UNE REPRÉSENTATION D'UNE ARCADE DENTAIRE**
ANALYSEVERFAHREN DER DARSTELLUNG EINER ZAHNREIHE
METHOD FOR ANALYSING A REPRESENTATION OF A DENTAL ARCH

(43) Date de publication de la demande: 31.07.2019
(73) Titulaire: Dental Monitoring, 75008 Paris (FR)
(72) Inventeur: SALAH, Philippe, BAGNOLET 93170 (FR); PELLISSARD, Thomas, 92110 CLICHY (FR)
(74) Mandataire: Nony

(56) Documents cités:
- EP-A1- 3 050 534
- WO-A1-2016/182551
- US-A1- 2007 129 991
- US-A1- 2015 006 192
- US-A1- 2017 061 087

## Description

### Domaine technique

La présente invention concerne le domaine de l'analyse de représentations d'arcades dentaires.

### Etat de la technique

Les scanners optiques ou "3D" permettent de créer des modèles tridimensionnels des surfaces de la bouche, mais ne permettent pas d'acquérir des informations sur les parties non visibles de la bouche. Les parties non visibles de la bouche comprennent en particulier les dents incluses, les racines des dents et les os maxillaires et mandibulaires.

Pour acquérir de telles informations, on procède classiquement à une acquisition tomographique, de préférence par tomographie informatisée à faisceau conique, en anglais CBCT pour *"cone beam computed tomography".* Une acquisition au moyen d'un appareil à rayons X classique est également possible, mais ne fournit pas toujours une résolution suffisante.

L'application de rayons X peut être nuisible pour la santé, en particulier si elle est répétée. Elle n'est donc pas pratiquée à des fins préventives. Autrement dit, les parties non visibles de la bouche ne sont généralement analysées que lorsque le patient se plaint de douleurs ou constate des phénomènes anormaux comme des dents trop « mobiles ».

Le diagnostic est donc tardif et le traitement long et complexe.

Il existe un besoin pour un procédé permettant d'évaluer la position et/ou la forme de parties non visibles de la bouche et qui ne pose pas les problèmes susmentionnés.

Le document EP 3 050 534 A décrit ansi un procédé de simulation de l'évolution d'un traitement orthodontique, le procédé permet également de prédire l'apparition d'une maladie parodontale.

Par ailleurs, chacun peut trouver un intérêt à prévoir l'évolution de son système dentaire, c'est-à-dire non seulement des dents visibles, mais également des dents incluses, des mâchoires ou du parodonte. Le seul examen des parties visibles de la bouche ne permet pas de prévoir avec précision une telle évolution.

Il existe donc également un besoin pour un procédé permettant d'anticiper l'évolution du système dentaire, indépendamment de tout diagnostic.

Enfin, l'orthodontiste ne disposant que d'une information limitée sur la configuration du système dentaire du patient peut être conduit à effectuer des actes inutiles ou préjudiciables. Par exemple, il peut demander au patient de porter un appareil orthodontique afin de modifier la position d'une dent qui, en l'absence de tout traitement, aurait naturellement évolué vers cette position.

Il existe donc un besoin permanent pour enrichir l'information mise à disposition de l'orthodontiste.

Un but de l'invention est de répondre, au moins partiellement, à ces besoins.

### Résumé de l'invention

L'objet de la présente invention est défini par le procédé de la revendication

Les revendications dépendantes fournissent des caractéristiques préférées du procédé selon l'invention.

La présente description présente également un procédé d'analyse d'une représentation dentaire d'une arcade dentaire d'un patient, dite « représentation dentaire d'analyse », procédé dans lequel la représentation dentaire d'analyse est soumise à un dispositif d'apprentissage profond, de préférence un réseau de neurones, afin de déterminer au moins une information dentaire relative à un objet dentaire associé à la représentation dentaire d'analyse et choisi parmi :
- une partie visible de l'arcade, notamment une couronne ou un ensemble de couronnes ou une arcade visible ; ou
- une partie non visible de ladite arcade, notamment une racine ou un ensemble de racines ou une ou plusieurs dents incluses.

Par « partie non visible », on entend une partie de l'arcade qui ne peut être vue en lumière visible, notamment à l'œil nu, et en particulier sans recours à un rayonnement X.

La représentation dentaire peut être en particulier une image ou un groupe d'images portant sur l'arcade dentaire du patient.

Un objet dentaire est dit « associé » à une représentation dentaire lorsque cet objet dentaire est représenté dans la représentation dentaire, par exemple une couronne, ou lorsqu'il lié à un élément représenté dans la représentation dentaire, par exemple une racine, généralement non visible, d'une couronne.

La présente description présente en particulier un procédé d'analyse d'une représentation dentaire d'analyse représentant, en plusieurs dimensions, une arcade dentaire d'un patient actuel, ledit procédé comportant les étapes suivantes :
1) création d'une base d'apprentissage comportant plus de 1000 structures dentaires historiques, chaque structure dentaire historique comportant :
   - une représentation dentaire historique représentant en plusieurs dimensions une arcade d'un patient historique, et
   - un descriptif historique contenant une valeur pour au moins un premier attribut relatif à un objet dentaire associé à ladite représentation dentaire historique,
2) entraînement d'au moins un dispositif d'apprentissage profond, de préférence un réseau de neurones, au moyen de la base d'apprentissage ;
3) soumission de la représentation dentaire d'analyse audit dispositif d'apprentissage profond de manière qu'il détermine, pour ladite représentation dentaire d'analyse, au moins une valeur pour ledit premier attribut.

Comme on le verra plus en détail dans la suite de la description, un tel procédé d'analyse permet avantageusement de reconnaître immédiatement des informations dentaires contenues dans la représentation dentaire d'analyse, mais qu'un orthodontiste aurait des difficultés ou ne pourrait pas identifier. Sa mise en œuvre contribue ainsi à la qualité du traitement.

Dans un mode de réalisation, à l'étape 3), on soumet au dispositif d'apprentissage profond non seulement la représentation dentaire d'analyse, mais également un descriptif d'analyse contenant une valeur pour au moins un deuxième attribut pour lequel les descriptifs historiques fournissent une valeur. La précision de l'analyse en est considérablement améliorée.

L' 'utilisation d'un tel procédé permet de contrôler la position et/ou de la forme d'une partie non visible de la bouche, et en particulier des racines des dents.

La description présente enfin :
- un programme d'ordinateur, et en particulier un applicatif spécialisé pour téléphone mobile, comprenant des instructions de code de programme pour l'exécution d'une ou plusieurs étapes d'un procédé, lorsque ledit programme est exécuté par un ordinateur,
- un support informatique sur lequel est enregistré un tel programme, par exemple une mémoire ou un CD-ROM.

### Définitions

Une représentation dentaire d'une arcade est un modèle numérique en plusieurs dimensions représentant tout ou partie de ladite arcade. La représentation dentaire peut être notamment une image en deux dimensions, un groupe d'images relatif à la même arcade, un modèle numérique tridimensionnel ou un hologramme.

Par "image", on entend une image en deux dimensions, comme une photographie ou une image extraite d'un film. Une image est formée de pixels.

Le groupe d'images peut comporter plusieurs images acquises au même moment et constituant par exemple une représentation en plusieurs morceaux de l'arcade dentaire. Le groupe d'images peut également comporter plusieurs images acquises à des instants différents, par exemple séparés par plus d'une semaine ou plus d'un mois. La représentation constitue ainsi une séquence d'images et fournit une information temporelle sur l'arcade dentaire. Elle peut être qualifiée de «représentation dynamique », ou de « représentation en quatre dimensions » de l'arcade.

On appelle « descriptif » d'une représentation dentaire, un ensemble de valeurs pour des attributs de cette représentation dentaire.

Une représentation dentaire et un descriptif de cette représentation dentaire constituent ensemble une « structure dentaire », ou « représentation dentaire enrichie ».

Une représentation dentaire, et en particulier une image ou un groupe d'images, est dite « historique » lorsqu'elle a été acquise dans le passé. Une structure dentaire est dite « historique » lorsqu'elle se réfère à une représentation dentaire historique. Les structures dentaires historiques sont placées dans la base d'apprentissage pour entrainer le dispositif d'apprentissage profond,

Chaque représentation dentaire peut être caractérisée par un descriptif fournissant un ensemble de valeurs propres à cette représentation dentaire, pour un ensemble d'attributs. Le nombre de valeurs possible pour un attribut n'est pas limité.

Un dispositif d'apprentissage profond est capable d'évaluer des valeurs d'attribut d'une représentation dentaire qui lui est soumise, c'est-à-dire d'une représentation dentaire d'analyse. A cet effet, il doit préalablement apprendre à analyser les représentations dentaires. On lui soumet donc une base d'apprentissage constituée d'un grand nombre de structures dentaires historiques, constituées chacune d'une représentation dentaire historique et d'un descriptif historique fournissant les valeurs des attributs pour la représentation dentaire historique, lesdites valeurs étant établies, par exemple manuellement, à partir de connaissances de l'homme de l'art.

Bien entendu, pour que le dispositif d'apprentissage profond puisse évaluer la valeur d'un attribut de la représentation dentaire d'analyse, les structures dentaires historiques doivent fournir des valeurs pour cet attribut ou pour des attributs équivalents.

Un « patient » est une personne pour laquelle une représentation dentaire a été acquise, indépendamment du fait que cette personne suive un traitement orthodontique ou non. On distingue le patient « actuel », à partir duquel est acquise la représentation dentaire d'analyse, et les patients historiques, à partir desquels sont acquises les représentations dentaires historiques de la base d'apprentissage.

Par « orthodontiste », on entend toute personne qualifiée pour prodiguer des soins dentaires, ce qui inclut également un dentiste.

Par « pièce orthodontique », on entend tout ou partie d'un appareil orthodontique.

Une pièce orthodontique peut être en particulier une gouttière orthodontique. Une telle gouttière s'étend de manière à suivre les dents successives de l'arcade sur laquelle elle est fixée. Elle définit une goulotte de forme générale en « U », dont la forme est déterminée pour assurer la fixation de la gouttière sur les dents, mais également en fonction d'un positionnement cible souhaité pour les dents. Plus précisément, la forme est déterminée de manière que, lorsque la gouttière est dans sa position de service, elle exerce des contraintes tendant à déplacer les dents traitées vers leur positionnement cible, ou à maintenir les dents dans ce positionnement cible.

La « position de service » est la position dans laquelle la pièce orthodontique est portée par le patient.

La « calibration » d'un appareil d'acquisition est constituée par l'ensemble des valeurs des paramètres de calibration. Un "paramètre de calibration" est un paramètre intrinsèque à l'appareil d'acquisition (à la différence de sa position et de son orientation) dont la valeur influence l'image acquise. De préférence, les paramètres de calibration sont choisis dans le groupe formé par l'ouverture de diaphragme, le temps d'exposition, la distance focale et la sensibilité.

Il faut interpréter "comprenant " ou "comportant " ou "présentant " de manière non restrictive, sauf indication contraire.

### Brève description des figures

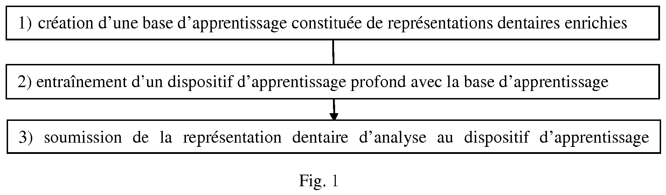

D'autres caractéristiques et avantages de l'invention apparaîtront encore à la lecture de la description détaillée qui va suivre et à l'examen du dessin annexé dans lequel la figure 1 représente, schématiquement, les différentes étapes d'un procédé d'analyse.

### Description détaillée

**A l'étape 1),** un ensemble de représentations dentaires historiques est collecté pour créer la base d'apprentissage.

De préférence, cet ensemble comporte plus de 1 000, plus de 5 000, plus de 10 000, de préférence plus de 30 000, de préférence plus de 50 000, de préférence plus de 100 000 représentations dentaires.

Chaque représentation dentaire historique est analysée, classiquement par un opérateur, afin d'établir le descriptif historique de cette représentation dentaire.

En particulier, pour chaque représentation dentaire collectée, un opérateur peut identifier les zones représentant une couronne, les numéros de dents et/ou les pathologies associées, les zones représentant une pièce orthodontique et les défauts éventuellement associés, par exemple le décollement éventuel des gouttières. Il affecte ainsi aux attributs de la représentation dentaire des valeurs d'attribut.

Par exemple, un orthodontiste est capable, à l'examen d'une représentation dentaire historique comportant la représentation d'une gouttière en service, par exemple d'une image, de déterminer la valeur de l'attribut « positionnement de la gouttière», c'est-à-dire d'établir si cette valeur doit être « gouttière correctement positionnée » ou « gouttière non correctement positionnée ». Cette valeur peut ainsi faire partie du descriptif historique de la représentation dentaire historique.

### Attribut

Un attribut peut porter sur un objet dentaire, c'est-à-dire relatif à l'appareil manducateur. Par exemple, un attribut peut être relatif à la position et/ou à la nature d'une couronne, d'une racine, d'un os alvéolaire, d'une arcade, de la langue, de la bouche, des lèvres, d'une mâchoire, de la gencive, de l'appareil manducteur dans son ensemble ou d'une pièce orthodontique portée par le patient.

Par exemple, il peut préciser les zones de dent dans la représentation dentaire, la position de la langue (par exemple « en retrait ») ou l'ouverture de la bouche du patient (par exemple « bouche ouverte » ou « bouche fermée ») ou la situation dentaire générale (par exemple « situation dentaire satisfaisante » ou « situation dentaire insatisfaisante », ou la présence d'une représentation d'un appareil orthodontique et/ou son état (par exemple appareil « intact », « cassé » ou « endommagé »), ou une information sur le patient (par exemple l'âge du patient ou relative à son environnement ou à ses habitudes, notamment alimentaires, ou sur le traitement qu'il suit ou qu'il a suivi dans le passé).

Un attribut de dent est de préférence choisi parmi un numéro de dent, un type de dent, dont les valeurs possible seraient par exemple « incisive », « canine » ou « molaire », un paramètre de forme de la dent, par exemple une largeur de dent, en particulier une largeur mésio-palatine, une épaisseur, une hauteur de couronne, un indice de déflexion en mésial et distal du bord incisif, ou un niveau d'abrasion, un paramètre d'apparence de la dent, en particulier un indice de translucidité ou un paramètre de couleur, un paramètre relatif à l'état de la dent, par exemple « abrasée », « cassée », « cariée » ou « appareillée » (c'est-à-dire en contact avec un appareil orthodontique), ou une combinaison de ces attributs. Un attribut de dent peut être également sa vitesse de déplacement en translation ou en rotation autour d'un axe de l'espace. Dans un mode de réalisation, des attributs de dents fournissent des valeurs pour les vitesses de déplacement en translation le long d'au moins trois axes formant un référentiel dans l'espace et pour les vitesses en rotation autour d'au moins trois axes formant un référentiel dans l'espace.

Dans un mode de réalisation particulièrement avantageux, le premier attribut est relatif à un objet dentaire non représentée sur la représentation dentaire considérée. En particulier, le premier attribut peut être relatif à une partie cachée de la dent, par exemple être relatif à la racine de la dent.

Pour un groupe d'images, des attributs peuvent être globaux pour le groupe, par exemple lorsqu'ils sont relatifs à la situation dentaire générale. Des attributs peuvent être propres à chaque image, comme l'instant d'acquisition de l'image. Des attributs peuvent enfin être propres à des parties de chaque image, comme la position des zones de dents représentées.

Des attributs de groupe peuvent être en particulier relatifs à la vitesse de déplacement d'une partie visible de la bouche, et en particulier de dents, ou relatif au traitement au cours duquel les images du groupe ont été acquises.

Un attribut peut également porter sur un autre objet qu'un objet dentaire, par exemple sur l'appareil d'acquisition de la représentation dentaire considérée. Par exemple, pour une image, un attribut peut être relatif à une position et/ou une orientation et/ou une calibration d'un appareil d'acquisition utilisé pour acquérir ladite image, par exemple un téléphone portable ou un scanner 3D. Il peut par exemple prendre les valeurs « photo de face », « photo de gauche » et « photo de droite ».

Un attribut peut également porter sur une propriété de la représentation dentaire, par exemple, être relatif à un identifiant des images d'un groupe, ou, pour une image, être relatif à la date d'acquisition de l'image, au groupe auquel appartient l'image, à la luminosité, au contraste ou à la netteté de l'image. Il peut par exemple prendre les valeurs « contraste insuffisant » et « contraste acceptable ».

Les attributs peuvent être également classés selon qu'ils concernent une partie seulement d'une représentation dentaire, ou « attribut spécifique », ou la représentation dentaire dans son ensemble, « attribut global ».

Par exemple, un attribut global peut être relatif à l'appareil d'acquisition utilisé lors de l'acquisition de la représentation dentaire ou au contexte de cette acquisition. Un attribut spécifique peut être relatif à une dent représentée, ou « attribut de dent ».

Au moins un attribut est relatif à un objet dentaire. Le premier attribut peut être notamment l'un quelconque des attributs décrits ci-dessus se rapportant à un objet dentaire.

Dans un mode de réalisation préféré, la détermination des descriptifs historiques est, au moins en partie, automatique.

De préférence, la valeur d'au moins un attribut d'une première représentation dentaire historique est déterminée au moyen de valeurs d'un ou plusieurs attributs d'une ou plusieurs représentations dentaires historiques acquises antérieurement. Par exemple, si deux représentations dentaires sont relatives au même patient, l'âge du patient renseigné dans l'attribut « âge » de la représentation dentaire antérieure permet de déduire l'âge du patient de la première représentation dentaire, en tenant compte du décalage temporel entre les instants d'acquisition des deux représentations dentaires historiques.

Dans un mode de réalisation, pour décrire une représentation dentaire historique d'une arcade dentaire d'un patient, on déforme un modèle tridimensionnel numérique initial de ladite arcade de manière à obtenir un modèle tridimensionnel numérique déformé présentant une concordance maximale avec ladite représentation dentaire historique. Le modèle tridimensionnel numérique initial peut être en particulier un scan optique 3D, par exemple effectué en début de traitement. L'acquisition de la représentation dentaire historique peut être par exemple postérieure de plus d'une semaine, un mois, ou deux mois de la réalisation du modèle tridimensionnel numérique initial.

On appelle « concordance » (« *match* » ou « *fit* » en anglais) entre deux objets une mesure de la différence entre ces deux objets. Une concordance est maximale (« *best fit* ») lorsqu'elle résulte d'une optimisation permettant de minimiser ladite différence.

Un modèle tridimensionnel numérique déformé présente une concordance maximale avec une représentation dentaire historique lorsque ce modèle a été choisi parmi plusieurs modèles parce qu'il permet une observation, par exemple une vue, présentant une concordance maximale avec ladite représentation dentaire historique, par exemple une image.

La recherche de la déformation optimale, c'est-à-dire conduisant au modèle tridimensionnel numérique déformé présentant une concordance maximale avec ladite représentation dentaire historique, se fait de préférence par des première et deuxième opérations.

Lors de la première opération, on recherche une observation, ou « vue », du modèle présentant une concordance maximale avec la représentation dentaire.

Lors de la deuxième opération, optionnelle mais préférée, on recherche une déformation supplémentaire du modèle, puis on reprend la première opération. Les deux opérations sont renouvelées jusqu'à obtenir un modèle tridimensionnel numérique déformé présentant une concordance maximale avec ladite représentation dentaire historique.

De préférence, la première opération et/ou la deuxième opération, de préférence la première opération et la deuxième opération mettent en œuvre une méthode métaheuristique, de préférence évolutionniste, de préférence un recuit simulé.

La méthode métaheuristique est de préférence choisie dans le groupe formé par
- les algorithmes évolutionnistes, de préférence choisie parmi:
   les stratégies d'évolution, les algorithmes génétiques, les algorithmes à évolution différentielle, les algorithmes à estimation de distribution, les systèmes immunitaires artificiels, la recomposition de chemin Shuffled Complex Evolution, le recuit simulé, les algorithmes de colonies de fourmis, les algorithmes d'optimisation par essaims particulaires, la recherche avec tabous, et la méthode GRASP ;
- l'algorithme du kangourou,
- la méthode de Fletcher et Powell,
- la méthode du bruitage,
- la tunnelisation stochastique,
- l'escalade de collines à recommencements aléatoires,
- la méthode de l'entropie croisée, et
- les méthodes hybrides entre les méthodes métaheuristiques citées ci-dessus.

Certaines valeurs d'attribut du modèle tridimensionnel numérique initial sont identiques dans le modèle tridimensionnel numérique déformé, et peuvent donc être automatiquement attribuées à la représentation dentaire. Par exemple, si les dents sont modélisées dans le modèle tridimensionnel numérique initial (segmentation du modèle) et que la déformation est un déplacement d'un ou plusieurs modèles de dents, les modèles de dents sont identifiables dans le modèle tridimensionnel numérique déformé, mais aussi, par conséquence, sur la représentation dentaire historique. Le modèle tridimensionnel numérique déformé a en effet été déterminé pour que la représentation dentaire historique puisse être observée sur lui. Les zones de dent identifiées sur le modèle tridimensionnel numérique initial peuvent alors être automatiquement identifiées sur la représentation dentaire historique.

Avantageusement, la déformation du modèle tridimensionnel numérique est possible lorsque la représentation dentaire historique est une photographie ou un film pris sans précaution particulière, par exemple avec un téléphone portable, comme décrit dans WO 2016 066651.

**A l'étape 2),** on entraîne un ou plusieurs dispositifs d'apprentissage profond au moyen de la base d'apprentissage.

Un dispositif d'apprentissage profond, en anglais « *deep learning* », est de préférence un réseau de neurones. Un réseau de neurones ou « réseau neuronal artificiel » est un ensemble d'algorithmes bien connu de l'homme de l'art.

Le réseau de neurones peut être en particulier choisi parmi :
- les réseaux spécialisés dans la classification d'images, appelés « CNN » (« Convolutional neural network »), par exemple
   - AlexNet (2012)
   - ZF Net (2013)
   - VGG Net (2014)
   - GoogleNet (2015)
   - Microsoft ResNet (2015)
   - Caffe: BAIR Reference CaffeNet, BAIR AlexNet
   - Torch:VGG_CNN_S,VGG_CNN_M,VGG_CNN_M_2048,VGG_CNN_M_10 24,VGG_CNN_M_128,VGG_CNN_F,VGG ILSVRC-2014 16-layer,VGG ILSVRC-2014 19-layer, Network-in-Network (Imagenet & CIFAR-10)
   - Google : Inception (V3, V4).
- les réseaux spécialisés dans la localisation, et détection d'objets dans une image, les Object Détection Network, par exemple:
   - R-CNN (2013)
   - SSD (Single Shot MultiBox Detector : Object Détection network), Faster R-CNN (Faster Region-based Convolutional Network method : Object Détection network)
   - Faster R-CNN (2015)
   - SSD (2015).

La liste ci-dessus n'est pas limitative.

L'entraînement d'un dispositif d'apprentissage profond sous la forme d'un réseau de neurones consiste classiquement à activer les neurones qui le constituent. L'interconnexion de ces neurones définit ensuite l'architecture du réseau. Plus précisément, on recherche les valeurs des paramètres qui, lorsqu'on soumet les représentations dentaires historiques de la base d'apprentissage au dispositif d'apprentissage profond paramétré avec lesdites valeurs, lui permettent de déterminer des valeurs d'attribut qui se rapprochent le plus possible des valeurs d'attribut des descriptifs historiques associés aux dites représentations dentaires historiques.

L'entrainement permet au dispositif d'apprentissage profond d'apprendre progressivement à reconnaître des motifs, en anglais « *patterns* », sur une représentation dentaire et à les associer à des valeurs d'attribut.

Après s'être entraîné avec la base d'apprentissage, le dispositif d'apprentissage profond peut ainsi déterminer des valeurs pour les attributs d'une représentation dentaire d'analyse, sans intervention de l'homme de l'art.

**A l'étape 3),** on soumet la représentation dentaire d'analyse du patient actuel au dispositif d'apprentissage profond. Le dispositif d'apprentissage profond détermine en conséquence au moins une valeur pour le premier attribut relatif à l'objet dentaire. Il enrichit ainsi le descriptif d'analyse de la représentation dentaire d'analyse. De préférence, le dispositif d'apprentissage profond détermine des probabilités pour les différentes valeurs possibles pour ledit premier attribut.

Dans un mode de réalisation, on détermine encore, à partir de la ou des valeurs d'attribut fournies par le dispositif d'apprentissage profond pour le premier attribut, une information dentaire relative audit objet dentaire, puis on transmet cette information dentaire à un opérateur.

L'information dentaire peut en particulier comporter une valeur du premier attribut de la représentation dentaire d'analyse ou une probabilité relative à une telle valeur.

L'information dentaire générée par le procédé d'analyse peut être dérivée d'une ou plusieurs valeurs d'attribut. Par exemple, elle peut être relative à la situation dentaire du patient, et en particulier relative à un risque d'occurrence d'une situation pathologique, relativement à une partie visible de la bouche, par exemple un risque de malocclusion, ou à une partie non visible de la bouche, comme défini par la présente invention, être relative à un risque de rhizalyse ou « résorption radiculo-dentaire ».

Par exemple, si un attribut des représentations dentaires historiques définit globalement, pour chaque représentation dentaire historique considérée dans son ensemble, si une situation dentaire « est pathologique » ou « n'est pas pathologique », l'information dentaire générée par le procédé d'analyse peut être une valeur de cet attribut pour la représentation dentaire d'analyse. Alternativement, cette information dentaire peut être déduite d'autres valeurs d'attributs équivalents, par exemple d'une valeur d'attribut de dent précisant qu'une dent « est cariée ».

Ces procédés sont particulièrement utiles pour l'évaluation de la position et/ou de la forme d'une partie non visible de l'appareil manducateur, et notamment de racines de dents, ou de dents incluses.

Par exemple, pour contrôler la rhizalyse, ce qui est un objectif de la présente invention, on construit une base d'apprentissage à partir de représentations dentaires historiques, par exemple des photos, sur lesquelles un orthodontiste identifie les dents ayant subi une rhizalyse, et renseigne le descriptif historique en conséquence.

Bien entendu, pour identifier ces dents sur une représentation dentaire historique, par exemple sur une image, l'orthodontiste peut avoir utilisé d'autres moyens que la seule image. En particulier, il peut avoir utilisé des informations acquises antérieurement et/ou postérieurement à l'acquisition de ladite image, par exemple plus de 1 semaine, 1 mois ou un an avant ou après l'acquisition de la représentation dentaire historique. En particulier, il peut avoir appris que l'image correspondait à un risque de rhizalyse lorsque la rhizalyse est apparue.

Il peut également avoir utilisé des clichés acquis par rayons X, par exemple par tomographie informatisée à faisceau conique.

Dans un mode de réalisation, la base d'apprentissage comprend des groupes d'images, acquises à des moments différents pour un même patient. Le descriptif historique d'un groupe peut indiquer, pour chaque image, la ou les dents ayant subi une rhizalyse, ainsi que la date d'acquisition de l'image et/ou les vitesses des déplacements des dents. Le dispositif d'apprentissage profond est alors capable d'intégrer non seulement l'influence de l'agencement des dents sur le risque de rhizalyse, mais également la vitesse de déplacement des dents.

La connaissance de l'évolution de la situation dentaire dans le temps améliore considérablement les résultats obtenus avec un procédé selon l'invention.

Par exemple, une situation dentaire qui, à l'examen d'une unique image d'analyse, apparaîtrait comme « non pathologique » peut être considérée comme « potentiellement pathologique» lorsque l'analyse du groupe d'images d'analyse fait apparaître une évolution qui, au regard de la base d'apprentissage, doit être considérée comme potentiellement pathologique.

Par ailleurs, certaines situations dentaires peuvent n'être évaluées correctement que par l'analyse d'un groupe d'images d'analyse permettant d'observer simultanément plusieurs régions de la bouche qu'une seule image ne permettrait pas de visualiser. Le groupe d'images d'analyse peut également comporter des images d'analyse acquises à des instants différents.

De manière générale, l'utilisation de groupes d'images comme représentations dentaires permet avantageusement d'accroître la quantité d'informations disponible.

### Exemple

Dans un mode de réalisation préféré de l'invention, les structures dentaires historiques de la base d'apprentissage comportent chacune une image acquise par tomographie informatisée à faisceau conique et un descriptif historique précisant le traitement dans le cadre duquel ladite image a été acquise, l'identification des types de dent représentées sur ladite image (par exemple « canines », « molaires »,...), les vitesses de déplacement de ces dents et des informations sur l'occurrence d'une rhizalyse dans le cadre de ce traitement.

Les attributs sont ainsi, par exemple,
- la nature du traitement suivi ;
- pour chaque dent représentée :
   - le type de dent ;
   - la vitesse en translation selon l'axe Ox), dans un référentiel de l'espace Oxyz),
   - la vitesse en translation selon l'axe Oy),
   - la vitesse en translation selon l'axe Oz),
   - la vitesse en rotation autour de l'axe Ox),
   - la vitesse en rotation autour de l'axe Oy),
   - la vitesse en rotation autour de l'axe Oz),
- occurrence d'une rhizalyse dans le cadre de ce traitement ?

Les vitesses de déplacement peuvent en particulier avoir été évaluées par analyse d'images acquises au moyen d'un scanner conventionnel.

L'image acquise par tomographie informatisée à faisceau conique est de préférence une image acquise en début de traitement.

Après avoir entrainé un dispositif d'apprentissage profond, de préférence un réseau de neurones, avec cette base d'apprentissage, on lui soumet la représentation dentaire d'analyse d'un patient, à savoir une image acquise par tomographie informatisée à faisceau conique, de préférence en début de traitement.

On lui soumet également le descriptif d'analyse incomplet de la représentation dentaire d'analyse, le descriptif d'analyse comporte des valeurs d'attribut pour au moins les attributs dont les valeurs constituent les descriptifs historiques des structures dentaires historiques, à l'exception de l'attribut « occurrence d'une rhizalyse dans le cadre de ce traitement ? ». Autrement dit, on indique au dispositif d'apprentissage profond quelles sont les valeurs pour les attributs suivants de la représentation dentaire d'analyse :
- traitement suivi ;
- pour chaque dent représentée :
   - type de dent ;
   - vitesse en translation selon l'axe Ox), dans un référentiel de l'espace Oxyz),
   - vitesse en translation selon l'axe Oy),
   - vitesse en translation selon l'axe Oz),
   - vitesse en rotation autour de l'axe Ox),
   - vitesse en rotation autour de l'axe Oy),
   - vitesse en rotation autour de l'axe Oz).

Le premier attribut est donc « occurrence d'une rhizalyse dans le cadre de ce traitement ? ».

A partir de cet ensemble d'informations, le dispositif d'apprentissage profond fournit une probabilité pour chacune des valeurs possibles du premier attribut, c'est-à-dire une probabilité pour que la valeur soit « oui » et une probabilité pour que la valeur soit « non ». Il est ainsi possible de déterminer une probabilité d'occurrence de la rhizalyse sans avoir à acquérir une nouvelle image par tomographie informatisée à faisceau conique.

L'évaluation d'un risque de rhizalyse ne constitue pas un diagnostic. Elle permet cependant de détecter des situations à risque. Pour établir un diagnostic, il reste nécessaire d'effectuer une acquisition tomographique. L'invention permet cependant de limiter considérablement de telles acquisitions tomographiques en détectant les situations à risque.

L'invention permet ainsi d'anticiper l'occurrence d'une rhizalyse.

Enfin, comme cela apparaît clairement à présent, l'invention permet à l'orthodontiste de bénéficier d'une connaissance approfondie de la situation dentaire de son patient, ce qui limite le risque qu'il effectue des actes inutiles ou préjudiciables.

Bien entendu, l'invention n'est pas limitée aux modes de réalisation décrits ci-dessus et représentés.

En particulier, le patient n'est pas limité à un être humain. Un procédé selon l'invention peut être utilisé pour un autre animal.

## Revendications

1. Procédé d'analyse d'une représentation dentaire d'analyse représentant, en plusieurs dimensions, une arcade dentaire d'un patient actuel, ledit procédé, exécuté par ordinateur, comportant les étapes suivantes :
1) création d'une base d'apprentissage comportant plus de 1 000 structures dentaires historiques, chaque structure dentaire historique comportant
- une représentation dentaire historique représentant en plusieurs dimensions une arcade d'un patient historique, et
- un descriptif historique comportant les attributs suivants :
∘ un traitement orthodontique dans le cadre duquel ladite représentation dentaire historique a été acquise
∘ une identification de dents représentées sur ladite représentation dentaire historique
∘ une vitesse de déplacement de ces dents
∘ une information relative à une occurrence d'une rhizalyse dans le cadre de ce traitement,
2) entraînement d'au moins un réseau de neurones au moyen de la base d'apprentissage, l'entrainement permettant au réseau de neurones d'apprendre progressivement à reconnaître des motifs, sur une représentation dentaire et à les associer à des valeurs d'attribut ;
3) soumission, audit réseau de neurones, de la représentation dentaire d'analyse et d'un descriptif d'analyse comportant des valeurs d'attributs pour au moins les attributs dont les valeurs constituent les descriptifs historiques des structures dentaires historiques, à l'exception de l'attribut d'information relative à une occurrence d'une rhizalyse, de manière que le réseau de neurones détermine, pour ladite représentation dentaire d'analyse, au moins une valeur pour l'attribut d'information relative à une occurrence d'une rhizalyse dans le cadre de ce traitement.

2. Procédé selon l'une quelconque des revendications précédentes, dans lequel la représentation dentaire d'analyse et les représentations dentaires historiques représentent, en au moins trois dimensions, ladite arcade dentaire.

3. Procédé selon l'une quelconque des revendications précédentes, dans lequel la représentation dentaire d'analyse et les représentations dentaires historiques
- sont toutes une image, ou
- sont toutes des groupes d'images, ou
- sont toutes des modèles numériques tridimensionnels, ou
- sont toutes des hologrammes.

4. Procédé selon l'une quelconque des revendications 1 et 2, dans lequel la représentation dentaire d'analyse et les représentations dentaires historiques comportent des groupes d'images acquises simultanément, ou acquises successivement, au moins deux desdites images acquises successivement ayant été acquises à plus d'un mois d'intervalle.

5. Procédé selon l'une quelconque des revendications précédentes, dans lequel on détermine au moins une valeur d'un attribut d'une première représentation dentaire historique au moyen de valeurs d'un ou plusieurs attributs d'une ou plusieurs représentations dentaires historiques acquises antérieurement ou postérieurement.

6. Procédé selon la revendication immédiatement précédente, dans lequel on détermine au moins une valeur d'un attribut d'une représentation dentaire historique au moyen de valeurs d'un ou plusieurs attributs d'un modèle tridimensionnel numérique déformé résultant d'une déformation d'un modèle tridimensionnel numérique initial généré au moyen d'un scanner optique.

7. Procédé selon la revendication immédiatement précédente, dans lequel on détermine ladite déformation pour que le modèle tridimensionnel numérique déformé présente une concordance maximale avec la représentation dentaire historique.

8. Procédé selon la revendication immédiatement précédente, dans lequel on recherche ladite concordance maximale
i) en recherchant une observation d'un modèle tridimensionnel numérique à tester, initialement choisi comme le modèle tridimensionnel numérique initial, qui présente la meilleure concordance avec la représentation dentaire historique, puis
ii) en déformant le modèle tridimensionnel numérique à tester, puis en reprenant à l'étape i) jusqu'à obtenir un modèle tridimensionnel numérique à tester permettant une observation qui présente une concordance maximale avec la représentation dentaire historique.

9. Procédé selon l'une quelconque des revendications précédentes, dans lequel les vitesses de déplacement sont évaluées par analyse d'images acquises au moyen d'un scanner.

10. Procédé selon la revendication 3, dans lequel la représentation dentaire d'analyse et les représentations dentaires historiques sont toutes une image acquise par tomographie informatisée à faisceau conique.

11. Procédé selon la revendication immédiatement précédente, dans lequel, à l'étape 3), l'image acquise par tomographie informatisée à faisceau conique est acquise en début de traitement.

12. Procédé selon la revendication 1, dans lequel, le descriptif d'analyse comporte pour chaque dent représentée un type de dent, et les vitesses en déplacement suivantes, dans un référentiel de l'espace Oxyz) : une vitesse en translation selon l'axe Ox), une vitesse en translation selon l'axe Oy), une vitesse en translation selon l'axe Oz), une vitesse en rotation selon l'axe Ox), une vitesse en rotation selon l'axe Oy), une vitesse en rotation selon l'axe Oz).

13. Procédé selon l'une quelconque des revendications précédentes, dans lequel le réseau de neurones fournit une probabilité pour chacune des valeurs possibles pour l'attribut d'information relative à l'occurrence d'une rhizalyse.

## Patentansprüche

1. Verfahren zur Analyse einer Analyse-Zahndarstellung, die einen Zahnbogen eines gegenwärtigen Patienten in mehreren Dimensionen darstellt, wobei das von einem Computer ausgeführte Verfahren die folgenden Schritte aufweist:
1) Erzeugung einer Lerndatenbank, die mehr als 1 000 frühere Zahnstrukturen aufweist, wobei jede frühere Zahnstruktur aufweist
- eine frühere Zahndarstellung, die einen früheren Bogen eines Patienten in mehreren Dimensionen darstellt, und
- ein früheres Deskriptiv, das die folgenden Attribute aufweist:
∘ eine kieferorthopädische Behandlung, in deren Rahmen die frühere Zahndarstellung erlangt wurde
∘ eine Erkennung von in der früheren Zahndarstellung dargestellten Zähnen
∘ eine Bewegungsgeschwindigkeit dieser Zähne
∘ eine Information bezüglich eines Auftretens einer Wurzelresorption im Rahmen dieser Behandlung,
2) Training mindestens eines neuronalen Netzes mittels der Lerndatenbank, wobei das Training es dem neuronalen Netz erlaubt, progressiv zu lernen, Muster auf einer Zahndarstellung zu erkennen und sie Attributwerten zuzuordnen;
3) Übermittlung an das neuronale Netz der Analyse-Zahndarstellung und eines Analyse-Deskriptivs, das Attributwerte für mindestens die Attribute aufweist, deren Werte die früheren Deskriptive der früheren Zahnstrukturen bilden, mit Ausnahme des Informationsattributs bezüglich eines Auftretens einer Wurzelresorption, damit das neuronale Netz für die Analyse-Zahndarstellung mindestens einen Wert für das Informationsattribut bezüglich eines Auftretens einer Wurzelresorption im Rahmen dieser Behandlung bestimmt.

2. Verfahren nach einem der vorhergehenden Ansprüche, wobei die Analyse-Zahndarstellung und die früheren Zahndarstellungen den Zahnbogen in mindestens drei Dimensionen darstellen.

3. Verfahren nach einem der vorhergehenden Ansprüche, wobei die Analyse-Zahndarstellung und die früheren Zahndarstellungen
- alle ein Bild sind, oder
- alle Gruppen von Bildern sind, oder
- alle dreidimensionale digitale Modelle sind, oder
- alle Hologramme sind.

4. Verfahren nach einem der Ansprüche 1 und 2, wobei die Analyse-Zahndarstellung und die früheren Zahndarstellungen Gruppen von gleichzeitig erfassten oder nacheinander erfassten Bildern aufweisen, wobei mindestens zwei der nacheinander erfassten Bilder in einem Zeitabstand von mehr als einem Monat erfasst wurden.

5. Verfahren nach einem der vorhergehenden Ansprüche, wobei mindestens ein Wert eines Attributs einer ersten früheren Zahndarstellung mittels Werten eines oder mehrerer Attribute einer oder mehrerer früheren Zahndarstellungen bestimmt wird, die vorher oder nachher erfasst werden.

6. Verfahren nach dem direkt vorhergehenden Anspruch, wobei mindestens ein Wert eines Attributs einer früheren Zahndarstellung mittels Werten eines oder mehrerer Attribute eines verformten digitalen dreidimensionalen Modells bestimmt wird, das aus einer Verformung eines ursprünglichen digitalen dreidimensionalen Modells resultiert, das mittels eines optischen Scanners erzeugt wird.

7. Verfahren nach dem direkt vorhergehenden Anspruch, wobei die Verformung so bestimmt wird, dass das verformte digitale dreidimensionale Modell eine maximale Übereinstimmung mit der früheren Zahndarstellung aufweist.

8. Verfahren nach dem direkt vorhergehenden Anspruch, wobei die maximale Übereinstimmung gesucht wird
i) indem eine Beobachtung eines zu testenden digitalen dreidimensionalen Modells gesucht wird, das ursprünglich als das ursprüngliche digitale dreidimensionale Modell gewählt wurde, das die beste Übereinstimmung mit der früheren Zahndarstellung aufweist, dann
ii) indem das zu testende digitale dreidimensionale Modell verformt wird, dann indem zu Schritt i) wieder aufgenommen wird, bis ein zu testendes digitales dreidimensionales Modell erhalten wird, das eine Beobachtung erlaubt, die eine maximale Übereinstimmung mit der früheren Zahndarstellung aufweist.

9. Verfahren nach einem der vorhergehenden Ansprüche, wobei die Bewegungsgeschwindigkeiten durch Analyse von Bildern bewertet werden, die mittels eines Scanners erfasst werden.

10. Verfahren nach Anspruch 3, wobei die Analyse-Zahndarstellung und die früheren Zahndarstellungen alle ein Bild sind, das durch Computertomographie mit kegelförmigem Strahl erfasst wird.

11. Verfahren nach dem direkt vorhergehenden Anspruch, wobei im Schritt 3) das durch Computertomographie mit kegelförmigem Strahl erfasste Bild zu Beginn der Behandlung erfasst wird.

12. Verfahren nach Anspruch 1, wobei das Analyse-Deskriptiv für jeden dargestellten Zahn einen Zahntyp und die folgenden Bewegungsgeschwindigkeiten in einem Koordinatensystem des Raums Oxyz) aufweist: eine Translationsgeschwindigkeit gemäß der Achse Ox), eine Translationsgeschwindigkeit gemäß der Achse Oy), eine Translationsgeschwindigkeit gemäß der Achse Oz), eine Drehgeschwindigkeit gemäß der Achse Ox), eine Drehgeschwindigkeit gemäß der Achse Oy), eine Drehgeschwindigkeit gemäß der Achse Oz).

13. Verfahren nach einem der vorhergehenden Ansprüche, wobei das neuronale Netz eine Wahrscheinlichkeit für jeden der möglichen Werte für das Informationsattribut bezüglich des Auftretens einer Wurzelresorption liefert.

## Claims

1. Method of analysis of a diagnostic dental representation showing a dental arch of a current patient in several dimensions, said method, executed by a computer, comprising the following steps:
1) creation of a learning base comprising more than 1,000 historical dental structures, each historical dental structure comprising
- a historical dental representation showing an arch of a historical patient in several dimensions, and
- a historical specification containing the following attributes
- an orthodontic treatment in the framework of which said historical dental representation has been acquired
- an identification of teeth shown on said historical dental representation
- a speed of displacement of these teeth
- an information on the occurrence of a rhizalyse in the framework of this treatment,
2) training of at least one neural network by means of the learning base, the training allowing the neural network to progressively learn to recognize patterns on a dental representation and to associate them with attribute values;
3) submission, to said neural network, of the diagnostic dental representation and of an analysis specification comprising attribute values for at least the attributes whose values constitute the historical specifications of the historical dental representations, except for the attribute of information on the occurrence of a rhizalyse in the framework of this treatment.

2. Method according to any one of the preceding claims, in which the diagnostic dental representation and the historical dental representations show, in at least three dimensions, said dental arch.

3. Method according to any one of the preceding claims, in which the diagnostic dental representation and the historical dental representations
- are all an image, or
- are all groups of images, or
- are all three-dimensional digital models, or
- are all holograms.

4. Method according to any one of claims 1 and 2, in which the diagnostic dental representation and the historical dental representations comprise groups of images acquired simultaneously, or acquired successively, at least two of said images acquired successively having been acquired at more than a one month interval.

5. Method according to any one of the preceding claims, in which at least one value of an attribute of a first historical dental representation is determined by means of values of one or more attributes of one or more historical dental representations acquired at an earlier or later date.

6. Method according to the immediately preceding claim, in which at least one value of an attribute of a historical dental representation is determined by means of values of one or more attributes of a deformed three-dimensional digital model resulting from a deformation of an initial three-dimensional digital model generated by means of an optical scanner.

7. Method according to the immediately preceding claim, in which said deformation is determined so that the deformed three-dimensional digital model exhibits a best match with the historical dental representation.

8. Method according to the immediately preceding claim, in which said best match is sought
i) by searching for an observation of a three-dimensional digital model to be tested, initially chosen as the initial three-dimensional digital model, which exhibits the best match with the historical dental representation, then
ii) by deforming the three-dimensional digital model to be tested, then re-starting from the step i) until a three-dimensional digital model to be tested is obtained allowing an observation which exhibits a best match with the historical dental representation.

9. Method according to any one of the preceding claims, in which the speeds of displacement are evaluated by analyzing images acquired by means of a scanner.

10. Method according to claim 3, in which the diagnostic dental representation and the historical dental representations are all an image acquired by cone-beam computed tomography.

11. Method according to the immediately preceding claim, in which, at step 3), the image acquired by cone-beam computed tomography is acquired at the beginning of the treatment.

12. Method according to claim 1, in which the historical specification comprises for each tooth shown a type of tooth, and the following speeds of displacement, in a reference frame of the space Oxyz) : a translational speed along the axis Ox), a translational speed along the axis Oy), a translational speed along the axis Oz), a rotational speed around the axis Ox), a rotational speed around the axis Oy), a rotational speed around the axis Oz).

13. Method according to any one of the preceding claims, in which the neural network supplies a probability for each of the possible values of information on the occurrence of a rhizalyse in the framework of this treatment.
